# EUROPÄISCHE PATENTANMELDUNG

(11) **EP 3 545 983 A1**
(43) Veröffentlichungstag der Anmeldung: **02.10.2019**
(21) Anmeldenummer: 18164553.2
(22) Anmeldetag: 28.03.2018
(51) Int. Cl.: A61M 1/10, A61M 1/12

(54) **BLUTPUMPE**

(71) Anmelder: Berlin Heart GmbH, 12247 Berlin (DE)
(72) Erfinder: Winterwerber, Dr. Kim Peter, 12357 Berlin (DE); Granegger, Dr. Marcus, 10781 Berlin (DE); Graichen, Dr. Kurt, 13189 Berlin (DE); Schmidt, Bodo, 14513 Teltow (DE); Thamsen, Dr. Bente, 10781 Berlin (DE); Lommel, Michael, 10179 Berlin (DE); Affeld, Prof. Klaus, 10587 Berlin (DE)
(74) Vertreter: Pfenning, Meinig & Partner mbB

(57) **Zusammenfassung**

Gegenstand der Anmeldung ist eine Blutpumpe mit einem Gehäuse und einem in dem Gehäuse angeordneten Rotor. Der Rotor ist dabei spindelförmig ausgebildet. Ferner folgt das Gehäuse zumindest in Teilen der spindelförmigen Aufweitung des Rotors. Auf diese Weise lässt sich eine besonders effiziente Blutpumpe kleinen Ausmaßes schaffen.

## Beschreibung

Gegenstand der vorliegenden Anmeldung ist eine Blutpumpe.

Im Stand der Technik sind zahlreiche Blutpumpen bekannt. Blutpumpen gemäß der vorliegenden Anmeldung werden bevorzugt als Ventricular Assist Devices (VAD) eingesetzt. Die VADs können entweder in den linken Ventrikel (LVAD), in den rechten Ventrikel (RVAD), oder jeweils eine Pumpe in den rechten und linken Ventrikel (BiVAD) geschoben werden. Unter anderem wird angestrebt, besonders kleine Blutpumpen zu schaffen, welche dennoch die gewünschten Pumpeigenschaften besitzen. Hierzu zählt zum einen das Fördern einer Blutmenge zwischen einem und zehn bzw. sieben bzw. sechs bzw. fünf Litern pro Minute sowie eine besonders blutschonende Förderung. Unter einer blutschonenden Förderung wird hierbei verstanden, dass zum einen eine Hämolyse des Blutes vermieden wird und zum anderen eine Thrombenbildung an den Pumpenbestandteilen vermieden wird.

Die Blutpumpen umfassen zumeist einen Rotor und sind entweder als radiale, diagonale oder axiale Pumpe ausgebildet. Unter einer radialen Pumpe wird hierbei eine Beaufschlagung mit einer im Wesentlichen radialen Geschwindigkeitskomponente verstanden, welche das Blut im Bereich des Auslasses einer Blutpumpe nach Außen "schleudert". Die meisten dieser Pumpen besitzen einen Rotor, welcher entweder breiter als lang ist, oder die Länge und die Breite sehr ähnlich sind. Unter einer axialen Pumpe wird hierbei verstanden, dass dem Blut eine primär axiale Vortriebsrichtung gegeben wird und der Blutdruck auf der stromabwärtigen Seite der Beschaufelung des Rotors erhöht wird. Viele dieser Pumpen besitzen einen Rotor, dessen Länge größer ist als die Breite. Bei der Lagerung der Rotoren kann auf verschiedene Konfigurationen zurückgegriffen werden. Beispielsweise können Magnetlager, hydrodynamische Lager oder mechanische Lager verwendet werden. Sämtliche Lager haben dabei Vor- und Nachteile. Eine Variante einer Blutpumpe ist beispielsweise in der US 9 265 870 gezeigt. Dabei handelt es sich um eine Axialpumpe mit einem im Wesentlichen transversalen Auslass.

Aufgabe der vorliegenden Anmeldung ist es, eine alternative Blutpumpe zur Verfügung zu stellen.

Die anmeldungsgemäße Blutpumpe umfasst ein Gehäuse mit einer Gehäuseachse, einem stromaufwärtigen Einlass und einem stromabwärtigen Auslass. Zwischen dem Einlass und dem Auslass ist ein Strömungsbereich ausgebildet. Das Gehäuse ist dabei derart ausgebildet, dass die Blutpumpe beispielsweise durch eine Öffnung im Apex in den rechten oder linken Ventrikel geschoben werden kann, so dass der Einlass selbst im Ventrikel angeordnet ist. Der Auslass ist derart angeordnet, dass dieser außerhalb des Ventrikels liegt und beispielsweise mittels einer Kanüle, einer Graftanordnung bzw. einer Kombination einer Kanüle und Graftanordnung mit einem Gefäß, wie beispielsweise der Aorta oder der Subklavia-Arterie, verbunden werden kann.

Innerhalb des Gehäuses ist ein vorzugsweise spindelförmiger Rotor mit einer Beschaufelung angeordnet. Der spindelförmige Rotor weist dabei einen stromaufwärtigen Einlassabschnitt, einen stromabwärtigen Auslassabschnitt und einen zwischen dem Ein- und dem Auslassabschnitt gelegenen Zylinderabschnitt auf. Der Zylinderabschnitt stellt hierbei vorzugsweise denjenigen Abschnitt des Rotors dar, an welchem die Rotornabe (d.h. der Rotor gemessen ohne die Beschaufelung) den größten Durchmesser besitzt. Ferner ist der Zylinderabschnitt derart platziert, dass dieser im Wesentlichen die Mitte zwischen dem stromabwärtigen Beginn und dem stromaufwärtigen Ende des Rotors beherbergt.

In einer Ausführungsform ist der spindelförmige Rotor stromabwärts zumindest teilweise mechanisch gelagert. In einer anderen Ausführungsform ist der spindelförmige Rotor stromaufwärts zumindest teilweise mechanisch gelagert. In einer weiteren Ausführungsform ist der spindelförmige Rotor sowohl stromabwärts als auch stromaufwärts mechanisch gelagert.

Der Rotor weist ferner eine sich vom stromaufwärtigen Einlassabschnitt zum Zylinderabschnitt hin erstreckende Laufradbeschaufelung auf. Die Laufradbeschaufelung kann beispielsweise aus einer, zwei, drei oder mehr Schaufeln bestehen und kann in einigen Ausführungsformen wendelförmig ausgestaltet sein. Bevorzugt besitzt die Laufradbeschaufelung eine erste Krümmungsrichtung (links- oder rechtsdrehend) und erstreckt sich vorzugsweise wendelartig entlang der Rotationsachse des Rotors. Die Laufradbeschaufelung zur Förderung des Blutes (Laufradbeschaufelung) ist dabei entweder ausschließlich am stromaufwärtigen Einlassabschnitt, ausschließlich im Zylinderabschnitt angeordnet oder erstreckt sich vom stromaufwärtigen Einlassabschnitt zum Zylinderabschnitt hin und ragt dabei bis zu einem Viertel, der Hälfte oder drei Viertel der Länge des Zylinderabschnitts entlang dem selbigen.

Ferner kann die Laufradbeschaufelung in einigen Ausführungsbeispielen derart beschaffen sein, dass der Pitch der Beschaufelung vom stromaufwärtigen Ende der Beschaufelung zum stromabwärtigen Ende der Beschaufelung hin steigt, d.h. die Steigung der Beschaufelung wird "flacher" oder auch die Länge einer Umdrehung der Beschaufelung entlang der Längsachse nimmt zu.

In einer Ausführungsform besitzt die Beschaufelung an ihrem stromabwärtigen Ende ein Profil, welches eine besondere Form aufweist. Unter einem symmetrischen Ende wird hierbei verstanden, dass eine stromaufwärtige Kante einer Schaufel im Bereich des stromabwärtigen Endes eine vergleichbare Länge mit der Länge einer stromabwärtigen Kante besitzt. Die beiden Kanten können dabei über ein Rechteckprofil, oder ein Rundprofil zusammengeführt werden. Ein Krümmungsradius des Endes kann also kleiner als eine zwischen den beiden vorgenannten Kanten ausgebildete Schaufelbreite sein. Alternativ oder zusätzlich kann das Ende Unstetigkeiten, Vertiefungen, Ausnehmungen oder ähnliches aufweisen.

In weiteren Ausführungsformen können im Strömungsbereich Vortexgeneratoren angeordnet werden. Die Vortexgeneratoren sind derart konfiguriert, dass Wirbel ausgebildet werden, um das Auswaschen des stromabwärtigen Lagers zu unterstützen. Beispielhafte Vortexgeneratoren können beispielsweise Rillen oder Erhöhungen im Strömungsbereich, insbesondere am stromabwärtigsten Bereich des Strömungsbereichs sein. Die Rillen können sich beispielsweise entlang eines Abschnitts der Wand der Spiralkammer bzw. Ringkammer bzw. Schneckenkammer erstrecken. Die Vortexgeneratoren können also in einigen Ausführungsformen erst stromabwärts der Beschaufelung, bzw. in der Spiral-, Ring- oder Schneckenkammer angeordnet sein. Dabei können die Vortexgeneratoren so beschaffen sein, dass die durch die Vortexgeneratoren hervorgerufenen Sekundärströmungen (im Vergleich zur Primärströmung) auf das stromabwärtige Lager ausgerichtet werden. Die Vortexgeneratoren können beispielsweise an der stromabwärtigen Wand angeordnete Erhöhungen oder Rillen sein, beispielsweise stegförmige Erhöhungen oder Rillen, welche von der Wand in Richtung oder bis zum stromabwärtigen Lagerstator verlaufen und die Spülung des stromabwärtigen Lagers begünstigen. Dabei können die Vortexgeneratoren geradlinig oder gekrümmt entlang der Wand in Richtung des Lagerstators laufen.

In einer weiteren, alternativen oder zusätzlichen Ausführungsform ist die stromaufwärtige Beschaufelung derart ausgebildet, dass sich im Betrieb der Pumpe eine stromaufwärtige Drallströmung ausbildet. Diese Drallströmung kann dergestalt sein, dass sich diese im Teillastbetrieb, d.h. bei reduziertem Fördervolumen (ausgehend vom für den Patienten festgelegten Volllastbetrieb), verstärkt. In einigen Ausführungsformen kann vorgesehen sein, dass das Gehäuse unter anderem im Bereich der Aufweitung Nuten oder Rillen aufweist, welche der Ausbildung der stromaufwärtigen Drallströmung entgegenwirken. In einigen Ausführungsbeispielen sind die Nuten oder Rillen orthogonal zur Drehachse, beispielsweise als rotationssymmetrische Ringnuten ausgebildet.

In einer weiteren Ausführungsform kann der Rotor ein Dämpfungsglied auf der Beschaufelung, wie beispielsweise einen Stützring, insbesondere einen geschlossenen Stützring aufweisen. Der Stützring ist in einigen Ausführungsbeispielen derart beschaffen, dass dieser eine Rückströmung über die Rotorschaufeln abschwächt.

In einer weiteren Ausführungsform kann die stromaufwärtige Kante der Beschaufelung einen Neigungswinkel von weniger als 90° zur Drehachse besitzen, vorzugsweise zwischen 75° und 15°, besonders vorzugsweise zwischen 60° und 25° liegen. Hierdurch wird der Abstand zur Lagerstelle vergrößert und die Pre-Rotation abgeschwächt.

Der Strömungsbereich umfasst ferner stromabwärts des Einlasses eine entlang der Gehäuseachse gelegene Auslasskammer, welche den unter einem Winkel zum Einlass gelegenen Auslass umfasst. Der transversale Winkel kann dabei gegenüber der Gehäuseachse beispielsweise um 20° bis 100°, vorzugsweise 70° bis 95°, geneigt sein. Die Auslasskammer kann ferner in einer Ausführungsform einen gegenüber dem stromaufwärtig gelegenen Teil des Gehäuses vergrößerten Querschnitt umfassen. Bei einer Vergrößerung des Querschnitts wird die Auslasskammer beispielsweise als Spiralkammer bezeichnet. Im Falle einer ausbleibenden Vergrößerung des Querschnitts im Bereich der Auslasskammer wird von einer Ringkammer gesprochen.

Das Gehäuse ist ferner derart ausgebildet, dass der Strömungsbereich des Gehäuses (der Querschnitt des Strömungsbereichs zur Längsachse der Pumpe ist im Wesentlichen kreis- oder ellipsenförmig) zwischen dem stromaufwärtigen Abschnitt und dem zylinderförmigen Abschnitt des Rotors eine Vergrößerung des Querschnitts erfährt. Das heißt, in einigen Ausführungsbeispielen folgt der Querschnitt des Strömungsbereichs des Gehäuses der Aufweitung des spindelförmigen Rotors vom stromaufwärtigen Einlassabschnitt zum Zylinderabschnitt hin. Dabei kann von der Öffnung des Gehäuses bis zu einem im Bereich des stromaufwärtigen Einlassabschnitts gelegenen Abschnitts des Rotors der Querschnitt des Strömungsbereichs gleichbleibend zylinderförmig sein. Anschließend weitet sich der Strömungsbereich auf und verläuft spätestens im Bereich des Zylinderabschnitts des Rotors zylinderförmig bis zur Auslasskammer.

Der zwischen dem Rotor und dem Gehäuse definierte Strömungsbereich, welcher der Blutförderung dient, kann auch als Netto-Strömungsbereich bezeichnet werden. Der Querschnitt des Netto-Strömungsbereichs ist dabei im Wesentlichen ringförmig (annular) und kann dabei dergestalt sein, dass dieser zwischen dem stromaufwärtigen Abschnitt und dem zylinderförmigen Abschnitt des Rotors eine Verringerung oder ein Gleichbleiben des Querschnitts erfährt. Die Querschnittsfläche des Netto-Strömungsbereichs kann beispielweise zwischen 40 und 100 mm^2 betragen, beispielsweise zwischen 50 und 70 mm^2 betragen.

Durch die hier beschriebene Pumpe wird eine Axialpumpe mit einem vorzugsweise in radialer Richtung ausströmenden Auslassgehäuse kombiniert. Aufgrund der mechanischen Lagerung und der Verwendung entsprechender Werkstoffe für die Lager, beispielsweise einem (CVD-) Diamant oder einem harten Carbid (siehe hierzu auch beispielsweise die EP 16 19 16 13.5, welche vollumfänglich zum Bestandteil dieser Anmeldung gemacht wird), kann auch bei hohen Drehzahlen und Kräften der Wärmeabtransport aus der Pumpe verbessert werden und die Pumpe kann über einen großen Drehzahl- und Flussbereich hin betrieben werden. Der große Drehzahl- und Flussbereich ermöglicht unter anderem eine physiologische Regelung der Pumpe. Ferner ist es hierdurch möglich, auch bei niedrigen Mittelflüssen, während der Diastole keine Rückflüsse durch die Pumpe zu bewirken. Im Falle eines Diamantlagers kann dieses beispielsweise eine Diamantkugel und eine korrespondierende Diamantkalotte umfassen. Alternativ kann das Lager eine Carbidkugel und eine korrespondierende Carbidkalotte besitzen. Hinsichtlich der Materialien wird nochmals auf die EP 16 19 16 13.5 verwiesen.

Weitere Ausführungsformen gehen unter anderem aus den Unteransprüchen hervor.

In einer Ausführungsform liegt der stromabwärtige Abschnitt des Rotors in der Auslasskammer. Aufgrund der Spindelform des Rotors wird das durch den zylinderförmigen Abschnitt des zur Auslasskammer hin transportierten Bluts in die Auslasskammer vorgetrieben, deren effektives Volumen sich aufgrund der Verjüngung des stromabwärtigen Abschnitts des Rotors in der Auslasskammer vergrößert. Das effektive Volumen wird durch das Volumen der Auslasskammer abzüglich des in diese reichenden Abschnitts des Rotors und ggf. eines stromabwärtigen Auslassstators gebildet.

In einer weiteren Ausführungsform umfasst das Gehäuse in der Auslasskammer einen koaxial zur Gehäuseachse verlaufenden und sich stromaufwärtig erstreckenden Auslassstator, welcher einen Teil des stromabwärtigen Lagers bildet. Dies ist insbesondere im Falle einer mechanischen stromabwärtigen Lagerung hilfreich. Bei dem stromabwärtigen Lager kann es sich ebenfalls um ein mechanisches Ball-Cup-Lager handeln. Aufgrund der Form des stromabwärtigen Auslassabschnitts des Rotors umfließt das Blut das mechanische Lager und spült dieses in ausreichendem Maße, um Thrombenbildung zu verhindern. Eine Ausführungsform eines Lagers kann beispielsweise der EP 16 19 16 13.5 entnommen werden.

In einer Ausführungsform können der Auslassstator und der stromabwärtige Auslassabschnitt des Rotors in der Ebene quer zur Rotationsachse korrespondierend oder im Wesentlichen spiegelsymmetrisch ausgebildet sein und bilden in Verbindung mit den Innenwänden der Auslasskammer eine Ring- oder Spiralkammer mit dem effektiven Volumen. Das effektive Volumen kann beispielsweise zwischen 1500 und 4000 mm^3 betragen, vorzugsweise zwischen 2000 und 2500 mm^3 betragen. Auf diese Weise wird eine besondere schonende Richtungsumkehr des Blutes von der axialen Förderrichtung zur radialen Auslassrichtung hin gewährleistet bei gleichzeitiger Spülung des mechanischen, stromabwärtigen Lagers.

In einer weiteren Ausführungsform umfasst die Pumpe einen Einlassstator, welcher mittels Streben am Gehäuse angeordnet ist. Unter Streben (einer Strebe, zwei Streben, drei Streben, vier Streben oder mehr Streben) wird hierbei, im Gegensatz zu Flügeln, verstanden, dass diese ein Verhältnis der großen und der kleinen Achse des Querschnitts von weniger als 4:1 (optional weniger als 3:1) besitzt, d.h. die große Achse ist vorzugsweise weniger als 4-mal so lang wie die kleine Achse, wobei die große Achse in der Richtung der Rotationsachse liegt. Mittels der Strebe wird der Einlassstator lediglich im Strömungsbereich gehalten, das Strömungsverhalten des Blutes jedoch nicht richtend verändert, wie beispielsweise im Falle eines Strömungs-Gleichrichters (Flow Straightener). Alternativ kann der Stator auch durch Flügel gehalten werden, welche neben der Halterung des Stators auch eine strömungsrichtende Funktion besitzen.

In einer weiteren Ausführungsform sind die den Einlassstator am Gehäuse befestigenden Streben außerhalb des Gehäuses angeordnet. Die Streben, beispielsweise zwei, drei, vier oder mehr Streben (alternativ: Flügel) bilden somit einen vor dem Einlass der Pumpe liegenden "Einlasskäfig". Die Anordnung der Streben als Käfig kann in einigen Ausführungsbeispielen unter anderem die Funktion besitzen, dass im Falle eines Ansaugereignisses, bei welchem der Einlass der Pumpe an eine Ventrikelwand angesogen wird, weiterhin ein Blutfluss in den Einlass möglich ist. Da die Streben eine Art Dom bzw. Einlasskäfig vor dem Einlass bilden, liegt die stromaufwärtige Seite des Domes zuerst an der Ventrikelwand an und die zwischen den Streben gebildeten Öffnungen vermögen weiterhin einen Transport des Bluts in den Einlass des Gehäuses. Das Verhältnis der Fläche der Streben fs zur Öffnungsgröße des Einlasses fe (d.h. der Querschnitt des Einlasses abzüglich der durch die Streben wegfallenden Fläche) kann in der Ebene quer zur Rotationsachse dabei fs:fe kleiner als 1:3, in einigen Ausführungsbeispielen kleiner als 1:4 bzw. 1:5 bzw. 1:6 sein (kleiner bedeutet also eine relative Zunahme der Einlassquerschnittsfläche im Vergleich zur Streben- bzw. Flügelquerschnittsfläche). Das heißt, dass die nicht durch die Streben abgedeckte Fläche der Öffnung deutlich größer ist als die durch die Streben überdeckte Fläche. Aufgrund der sich aus dem Einlass hervorschiebenden Streben wird jedoch das Ansaugen vermieden. Das Verhältnis fs:fe sollte jedoch größer als 1:1000, vorzugsweise größer als 1:200 sein. Der Einlasskäfig kann sich in einigen Ausführungsbeispielen zwischen 2 bzw. 3 und 10 bzw. 8 mm aus der Einlassebene in stromaufwärtiger Richtung erstrecken.

Ferner wird durch das Anordnen der Streben außerhalb des Einlasses des Gehäuses bewirkt, dass das Gehäuse insgesamt kürzer ausgebildet sein kann. Da die Streben den Stator am Gehäuse befestigen, kann der Stator stromaufwärtiger angeordnet werden und somit auch der Rotor stromaufwärtiger befestigt werden. Hierbei bietet es sich insbesondere an, zwischen dem stromaufwärtigen Einlassabschnitt des Rotors und dem stromabwärtigen Ende des Einlassstators ein mechanisches Lager anzuordnen. Dies kann beispielsweise ebenfalls als Ball-Cup-Lager ausgebildet sein.

In einer weiteren Ausführungsform besitzt der stromabwärtige Auslassabschnitt eine Spülbeschaufelung bzw. eine Rillenstruktur, um das Blut zu einer torusförmigen Sekundärströmung im Auslassgehäuse anzuregen. Bei der Verwendung einer Spülbeschaufelung oder einer Rillenstruktur kann diese beispielsweise keine Krümmung aufweisen oder in einer der ersten Krümmungsrichtung entgegengesetzten oder gleichgerichteten Krümmungsrichtung ausgebildet sein.

Dabei ist in einigen Ausführungsformen vorgesehen, dass die Spülbeschaufelung bzw. die Rillenstruktur ausschließlich am stromabwärtigen Abschnitt des spindelförmigen Rotors angeordnet ist.

Die Spindelform des Rotors kann beispielsweise derart hergestellt werden, indem der stromabwärtige Abschnitt bzw. stromaufwärtige Abschnitt des spindelförmigen Rotors sich flaschenhalsartig zum Zylinderabschnitt hin aufweitet bzw. kegelförmig oder glockenförmig ist.

In einer weiteren Ausführungsform besitzt der Rotor eine diamantartige Beschichtung, beispielsweise eine "diamond like carbon" (DLC) Beschichtung. Auf diese Weise kann der metallische Werkstoff des Rotors, des Gehäuses, des Einlassstators oder des Auslassstators in seiner jeweiligen Hämokompatibilität weiter verbessert werden. Hierbei sei erwähnt, dass die Beschichtung der Blutkontaktflächen der Pumpe mit einer diamantartigen Schicht einen eigenständigen Teil der Anmeldung bildet und eigenständig, beispielsweise im Rahmen von Teilanmeldungen weiterverfolgt werden kann. Als Alternative zu der Beschichtung mit DLC kann auch nanokristalliner Diamant gewählt werden.

Der stromabwärtige Abschnitt schließt sich direkt an den zylinderförmigen Abschnitt an und verjüngt sich dabei stromabwärts.

In einer weiteren Ausführungsform ist in/an der Wand des Gehäuses beispielsweise im Bereich des stromaufwärtigen Lagers oder stromauf des stromaufwärtigen Lagers ein, mehr als ein Drucksensor oder andere Sensoren zur Messung eines Ventrikeldrucks im Strömungsbereich angeordnet. Hierdurch wird die Ventrikeldruckmessung vereinfacht. Als Drucksensor können beispielsweise ein MEMS oder membranbasierte Sensoren eingesetzt werden.

Insbesondere bei der Verwendung von mechanischen Lagern kann vorgesehen sein, dass eine Justiervorrichtung zur feinmechanischen Einstellung der Lager vorhanden ist. So kann beispielsweise ein stromabwärtiges mechanisches Lager mittels einer von der stromabwärtigen Seite der Auslasskammer her eingebrachten Schraube weiter vorgespannt werden. Das stromaufwärtige und/oder das stromabwärtige Lager kann beispielsweise in der Ebene des Lagers über einen oder mehrere Exzenter justiert werden. Auf diese Weise kann eine gute Ausrichtung der Statorteile des Lagers gewährleistet werden. Eine weitere Variante ist eine Axialverstellung über Feingewinde. Die Justierung der Lagerspalte kann auch durch eine außerhalb der Pumpe befindliche Einrichtung erfolgen.

In einer weiteren Ausführungsform wird die Ring- oder Spiralkammer durch zwei aufeinanderliegende Komponenten gebildet. Dabei kann eine stromaufwärtige Komponente auch einen stromabwärtigen Teil des Einlasses umfassen und in einigen Ausführungsbeispielen einteilig ausgebildet sein, d.h. der Teil des Einlasses und der Ring- bzw. Spiralkammer der stromaufwärtigen Komponente weisen keine Schweißnähte oder andere stoffschlüssige Verbindungen auf. Eine stromabwärtige Komponente, welche die Ring- oder Spiralkammer formt, wird in einigen Ausführungsbeispielen mittels Flächenpressung auf die stromaufwärtige Komponente gedrückt, so dass die Ring- und Spiralkammer nach Außen fluiddicht ist.

In einigen Ausführungsformen weisen sowohl die stromaufwärtige als auch die stromabwärtige Komponente Anpressflächen auf, welche aufeinandergelegt bzw. -gepresst werden können, beispielweise mittels Schrauben, Stiften, Bolzen und/oder Klebstoffen oder Schweißnähten.

In einigen Ausführungsformen sind die Anpressflächen derart ausgebildet, dass bei aufeinander gepressten Komponenten mindestens ein die Ringkammer umlaufender Kragen gebildet ist. Der Kragen kann dabei entweder in der stromaufwärtigen Komponente oder der stromabwärtigen Komponente angeordnet sein oder durch zwei Kragen der beiden Komponenten gebildet sein. Der Kragen verringert die Auflagefläche zwischen den Komponenten, so dass der Anpressdruck erhöht wird und die Dichtigkeit verbessert wird.

Ähnlich einem, die Ring- bzw. Spiralkammer umlaufenden Kragen, können die zwei Komponenten auch einen weiteren im Bereich der Außenkonturen der Komponenten angeordneten Kragen umfassen. Hierdurch wird eine Dichtigkeit gegenüber dem Körper verbessert, in welchen die Pumpe implantiert wird. Im Falle eines im Bereich der Außenkonturen und im Bereich der Ring- bzw. Spiralkammer angeordneten Kragens wird, in radialer Richtung betrachtet, zwischen den beiden Kragen ein Hohlraum gebildet. In diesem können entweder andere Pumpenkomponenten, wie z.B. Pumpenelektronik, angeordnet werden, und/oder der Hohlraum kann Bohrungen für die Schrauben (bzw. andere oben genannte Verbindungselemente) aufweisen, weiche zur kraft- und/oder formschlüssigen Verbindung der beiden Komponenten eingebracht werden. Die Verbindung der beiden Komponenten mittels der Kragen kann dabei auch in anderen Pumpen zum Einsatz kommen und ist nicht auf die in dieser Anmeldung offenbarte Pumpe beschränkt. Die Anmelderin behält sich das Recht auf die Kragen gerichtete Teilanmeldungen vor.

Weitere Ausführungsbeispiele können den Figuren entnommen werden.

Es zeigen:
- Figur 1: eine Pumpe in der Außenansicht;
- Figur 2: die Pumpe der Fig. 1 im Längsschnitt;
- Figur 3: eine alternative Pumpe im Längsschnitt;
- Figur 4: eine weitere alternative Pumpe im Längsschnitt;
- Figur 5: eine weitere Pumpe in einer transparenten Ansicht mit einem Einlasskäfig;
- Figur 6 A, B: ein Ausführungsbeispiel einer Pumpe mit mindestens einer stromauf- und einer stromabwärtigen Komponente;
- Figur 7 A-C: verschiedene Ausführungsformen eines stromabwärtigen Beschaufelungsprofils;
- Figur 8: Profil eines stromabwärtigen Abschnitts eines Rotors für eine Pumpe;
- Figur 9: eine weitere Ausführungsform einer Pumpe.

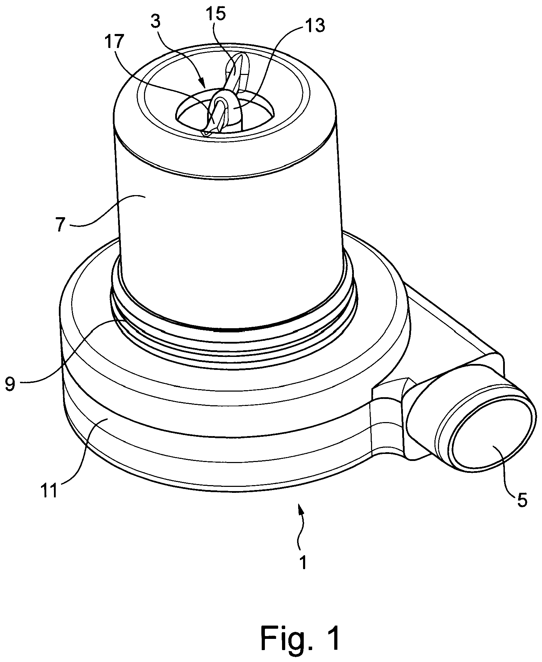

In der Fig. 1 wird eine Ausführungsform der anmeldungsgemäßen Blutpumpe in der Außenansicht gezeigt. Die Blutpumpe 1 besitzt einen Einlass 3 sowie einen transversalen Auslass 5, wobei der Einlass 3 und der Auslass 5 durch einen Strömungsbereich miteinander verbunden sind. Vom Einlass 3 betrachtet erstreckt sich ein Einlassrohr 7, an dessen unterem Ende ein Konnektor 9 zum Anbinden an einen Nahtring, welcher an einem Apex des Herzens befestigt ist, geeignet ist. Im Bereich des Nahtrings weitet sich der Körper der Pumpe auf und schafft Platz für das Außengehäuse 11 der Auslasskammer. Im Bereich des Einlasses ist der Stator 13 zu erkennen, welcher mittels zweiter Streben 15 und 17 im Bereich des Einlasses gehalten wird. Der Außendurchmesser des Pumpenrohrs 7 kann beispielsweise zwischen 1 und 3 cm betragen. Das Außenrohr 7 ist dabei in einigen Ausführungsbeispielen der Teil der Pumpe, welcher durch den Apex hindurch in den Ventrikel ragt.

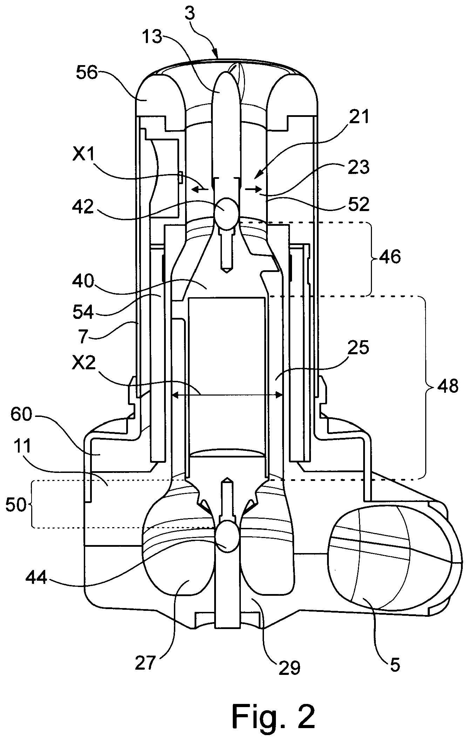

In der Fig. 2 ist der Querschnitt durch die in der Fig. 1 dargestellte Pumpe gezeigt. Wie bereits in der Fig. 1 erläutert, befindet sich zwischen dem Einlass 3 und dem Auslass 5 ein Strömungsbereich 21. Dieser weist im stromaufwärtigen Bereich 23 einen zylinderförmigen Querschnitt mit einem Durchmesser X1 auf. In einem stromabwärtigeren Abschnitt 25 besitzt der Strömungsbereich einen Durchmesser X2, welcher größer als der Durchmesser X1 des Abschnitts 23 ist. Vom Abschnitt 25 führt der Strömungsbereich in den Abschnitt 27, welcher durch eine kreisförmige oder spiralförmige Aufweitung gegeben ist. Der Durchmesser ist hierbei aufgrund des Auslasses 5 schwierig zu bestimmen. An seiner geringsten Stelle, kann der Durchmesser des Abschnitts 27 jedoch gleich oder größer als der Durchmesser X2 sein. Von der Öffnung des Einlasses 3 her erstreckt sich der Stator 13 in den Einlass 3 der Pumpe hinein. Vom stromabwärtigen Ende der Pumpe erstreckt sich ein Stator 29 in die Auslasskammer. Zwischen den beiden Statoren ist der Rotor 40 angeordnet. Der Rotor 40 ist hierbei durch ein erstes mechanisches Lager 42 und ein zweites mechanisches Lager 44 gehalten. Dabei kann es sich bei dem Lager beispielsweise um ein Ball-Cup-Bearing handeln, wobei sowohl der Ball als auch der Cup durch ein hartes Material, wie beispielsweise ein Diamant (beispielweise ein CVD-hergestellter Diamant), oder ein Siliziumcarbid oder eine andere Keramik gegeben sein kann. Details zu möglichen Lageranordnungen im Falle eines mechanischen Lagers können beispielsweise der Anmeldung EP 16191613 entnommen werden. Der sich zwischen den Lagerstellen 42 und 44 erstreckende Rotor 40 besitzt einen stromaufwärtigen Abschnitt 46, einen zylinderförmigen Abschnitt 48 sowie einen stromabwärtigen Abschnitt 50. Der stromabwärtige Abschnitt 50 und der stromaufwärtige Abschnitt 46 besitzen eine zum zylinderförmigen Abschnitt hin sich aufweitende Form, ähnlich einem Flaschenhals. Alternativ können die Abschnitte 46 und/oder 50 auch glockenförmig oder kegelförmig ausgebildet sein.

In seinem geringsten Durchmesser besitzt der stromaufwärtige Abschnitt 46 eine Ausnehmung, in welcher der Ball des Ball-Cup-Bearings gehalten werden kann. Von diesem Punkt aus weitet sich der stromaufwärtige Abschnitt 46 auf bis zum zylinderförmigen Abschnitt 48. Der zylinderförmige Abschnitt 48 weist dabei den größten Durchmesser des Rotors auf. Der sich direkt an den zylinderförmigen Abschnitt 48 anschließende stromabwärtige Abschnitt 50 weist ebenfalls eine Flaschenhalsform auf. Dabei reduziert sich der Querschnitt des Rotors zum stromabwärtigen Ende hin und der Rotor schließt beispielsweise mit dem mechanischen Lager 44 ab. Der zylinderförmige Abschnitt 48 kann beispielsweise hohl ausgebildet sein. In diesem Abschnitt können beispielsweise Permanentmagnete angeordnet werden, mittels welcher der Rotor entlang der zwischen den Lagern 42 und 44 ausgebildeten Rotationsachse in Rotation versetzt wird. In dem hier gezeigten Ausführungsbeispiel ist die Gehäuseachse koaxial zur Rotationsachse des Rotors. Der Motor zum Antrieb des Rotors befindet sich außerhalb der inneren Gehäusewand 52 des Gehäuserohrs 7. Der Motor 54 ist ein Mehrphasenelektromotor und ist derart konfiguriert, dass dieser den Rotor 40 auf bis zu 15000 Umdrehungen pro Minute rotieren kann. Die im zylinderförmigen Abschnitt angeordneten Motormagnete können beispielsweise einen Durchmesser von ca. 3 bis 10 mm, insbesondere von 8 bis 10 mm, und eine Höhe von 0,5 bis 2 cm, insbesondere 1 bis 2 cm, besitzen. Der Motor 54 ist derart im Gehäuse angeordnet, dass dieser zumindest teilweise innerhalb des Ventrikels angeordnet wird und/oder zumindest einen Teilbereich des zylinderförmigen Abschnitts 48 des Rotors koaxial umläuft. Auf diese Weise wird eine kurze, effiziente Axialpumpe konstruiert.

Der Durchmesser X2 des Abschnitts 25 beträgt beispielsweise 15 mm. Aufgrund des Durchmessers des Rotors von beispielsweise 10 bis 12 mm kann selbst bei vergleichsweise niedrigen Drehzahlen von 6000 bis 9000 U/min. bei einer Blutförderung von 5 l/min. und 80 mmHg eine gut blutverträgliche Pumpe bereitgestellt werden.

Das Gehäuserohr 7 schließt das Gehäuse derart ab, dass der Motor 54 nicht mit dem Blut in Kontakt kommt. Um das Einsetzen des Stators 13 zu vereinfachen, kann beispielsweise ein stromaufwärtiger Abschnitt 56 als eigenes Bauteil ausgebildet werden. Dieses umfasst dann die Innenwand des Abschnitts 23, den Stator 13 sowie die Streben 15 und 17, welche entlang der Gehäuseachse ein Maß von 2 mm und quer hierzu ein Maß von 1 mm besitzen. Durch diese Ellipsenform bieten die Streben 15 und 17 einen geringen Strömungswiderstand und werden lediglich umflossen. Eine Strömungs-Gleichrichtung findet hierdurch - falls überhaupt - nur unwesentlich statt. Die Streben 15 und 17 unterscheiden sich von strömungsbegradigenden Statorflügeln.

Zwischen dem Rohr 7 und dem äußeren Gehäuse 11 der Auslasskammer befindet sich ein Hohlraum 60, in welchem die Pumpenelektronik angeordnet werden kann. In diesem Bereich kann auch ein Ende einer Driveline aus dem Pumpengehäuse austreten, welche die Elektronik und den Motor mit der benötigten Energie versorgen kann. Zudem können über die Driveline auch die Ansteuersignale für die Pumpe gesendet werden, oder Sensorsignale aus der Pumpe zu einem Controller geleitet werden. Insgesamt beträgt die Länge der Pumpe entlang der Gehäuseachse beispielsweise 2 bis 5 cm (bei pädiatrischen Anwendungen) bzw. 4 bis 10 cm, insbesondere 7 bis 10 cm (bei Pumpen für Erwachsene).

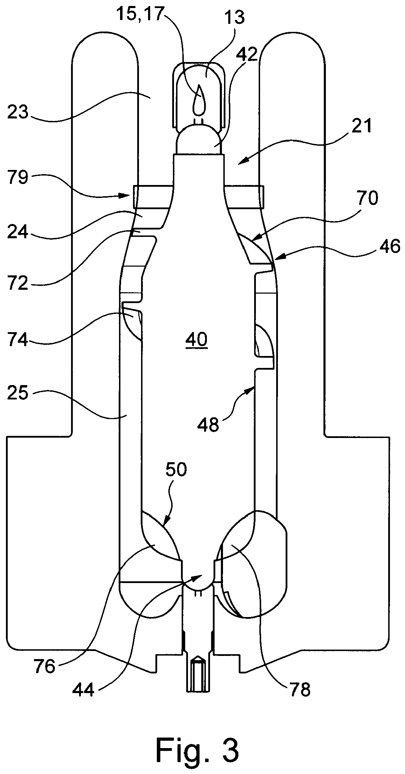

Ein schematischer Querschnitt einer alternativen Pumpe ist in der Fig. 3 dargestellt. Mit der Pumpe 1 vergleichbare Bauteile haben dabei die gleichen Bezugszeichen. In der Fig. 3 ist unter anderem der Rotor 40 gezeigt und die auf diesem angeordneten Laufradschaufeln 70, 72 und 74. Die Schaufeln erstrecken sich dabei in dem Abschnitt 24, in welchem sich der Querschnitt des Strömungsbereichs vom zylinderförmigen Bereich 23 zum zylinderförmigen Bereich 25 hin stetig aufweitet. Die Aufweitung erfolgt dabei korrespondierend zur Aufweitung des stromaufwärtigen Abschnitts 46 des Rotors. Allerdings ist die Schaufelhöhe senkrecht zur Gehäuseachse im stromaufwärtigen Abschnitt höher als die Schaufelhöhe am stromabwärtigen Ende der Beschaufelung.
Der Durchmesser der Nabe im einlassseitigen Rotorabschnitt kann beispielsweise in stromabwärtiger Richtung von 3 auf 11 mm aufgeweitet sein. Die Schaufelhöhe kann beispielsweise am stromaufwärtigen Ende zunächst 2 mm und am stromabwärtigen Ende der Beschaufelung 1,5 mm betragen. In anderen Ausführungsbeispielen ist jedoch vorgesehen, dass die Höhe der Beschaufelung konstant bleibt. Anhand der Fig. 3 kann zudem erkannt werden, dass der Steigungswinkel der Beschaufelung 70, 72, 74 gegenüber der Gehäuseachse in stromabwärtiger Richtung abnimmt. Das heißt, die zunächst steilen Schaufeln werden in Richtung des Auslasses hin weniger steil und sorgen für ein verbessertes Ausströmen des Blutes zur Auslasskammer 27 hin, so dass die Strömung entlang des Rotors verzögert bzw. der Druck entlang des Rotors aufgebaut wird. Anhand der Fig. 3 ist zudem erkennbar, dass die Laufradschaufeln 70, 72 und 74 sich lediglich über einen Teilabschnitt des Rotors erstrecken. Dabei ist in einigen Ausführungsbeispielen vorgesehen, dass sich die Laufradschaufeln lediglich im stromaufwärtigen ersten und zweiten Drittel der Rotorlänge befinden. In anderen Ausführungsbeispielen erstreckt sich die Beschaufelung lediglich bis zur Hälfte der Länge des Rotors. Anhand der Fig. 3 ist zudem erkennbar, dass die Streben 15 und 17 im Wesentlichen innerhalb des Einlasses liegen.

In der Fig. 3 ist zudem eine Variante des stromabwärtigen Auslassabschnitts des Rotors dargestellt. So weist der stromabwärtige Abschnitt 50 eine Vielzahl von Spülschaufeln 76 und 78 auf, welche sich vom stromabwärtigen Ende des zylinderförmigen Abschnitts bis zum stromabwärtigen Ende des Auslassabschnitts des Rotors hin erstrecken, entgegen der Krümmungsrichtung der Laufradschaufeln gekrümmt sind und einen verbesserten Blutfluss zur Lageranordnung 44 ermöglichen. Auf diese Weise wird das Lager 44 besser gespült und eine Thrombenbildung verhindert. Obgleich der stromabwärtige Abschnitt des Rotors Spülschaufeln umfasst, besitzt der Ball des Lagers, welcher am Rotor befestigt ist, keinerlei Strukturierung, sondern ist im Wesentlichen eine sphärische Oberfläche. Im Übergangsbereich zwischen dem zylindrischen Abschnitt 23 und dem sich aufweitenden Abschnitt 24 ist eine Ringnut 79 angeordnet, welche die Pre-Rotation abschwächen kann. Obgleich hier nur eine umlaufende Ringnut dargestellt ist, können mehrere, d.h. mehr als eine Ringnut, beispielsweise zwei oder drei parallele Ringnuten im Abschnitt 23 und/oder 24 angeordnet sein.

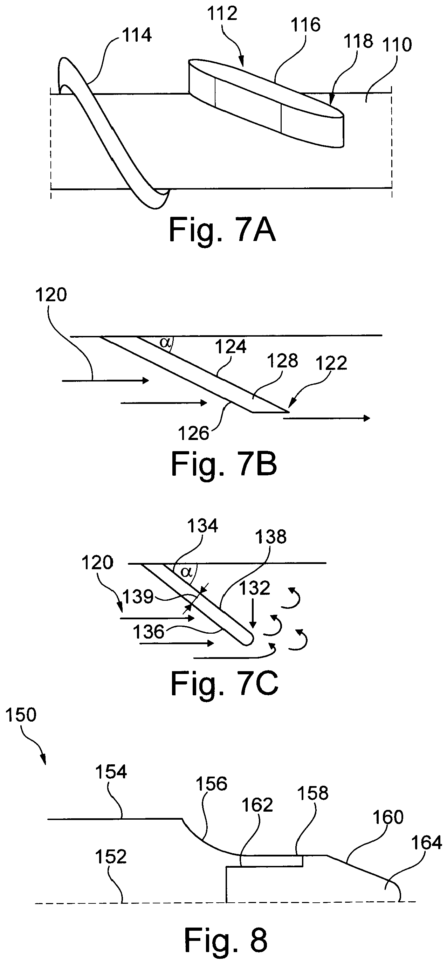

In den hier vorgestellten Ausführungsbeispielen und -formen ist die Laufradbeschaufelung 70 oftmals als dünnes Profil dargestellt. D.h. dass die Dicke der Schaufel in einer Ebene quer zur Rotationsachse deutlich geringer ist als der Abstand zwischen zwei benachbarten Laufradschaufeln 72 und 74. In anderen Ausführungsformen kann die Beschaufelung jedoch deutlich dicker ausgeführt sein. Beispielhaft sei hier die Beschaufelung der Fig. 7 der US 8007254 verwiesen: Der dort dargestellte Rotor besitzt Laufradschaufeln, welche deutlich dicker als der Abstand zweier benachbarter Laufradschaufeln sind. Auch eine derartige Beschaufelung kann bei den hier dargestellten Pumpen und Rotoren zur Anwendung kommen.

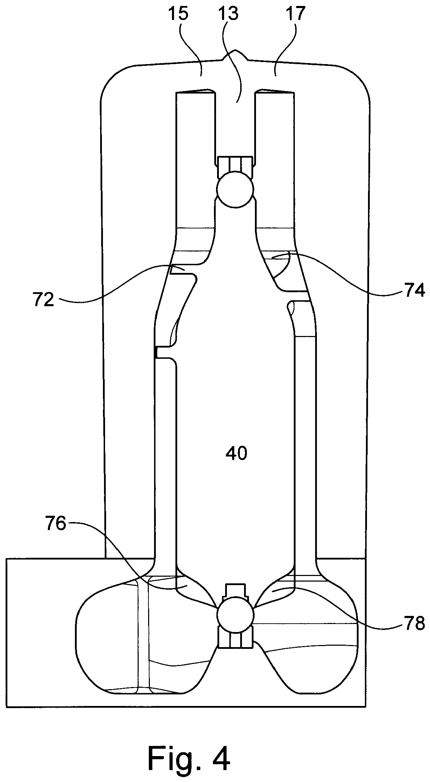

Eine weitere Variante der Pumpe ist in der Fig. 4 dargestellt. Die Fig. 4 unterscheidet sich dabei von der Fig. 3 dadurch, dass die Streben 15 und 17 weiter in Richtung des Einlasses versetzt sind und im Wesentlichen plan mit der Einlassöffnung beginnen. Die Länge der Streben entlang der Gehäuseachse ist lediglich dreimal so lang ist wie die maximale Breite quer zur Gehäuseachse. Alternativ zu den Spülschaufeln weist der Abschnitt 50 Rillen 78 auf, welche ohne Krümmung in Richtung der Lageranordnung 44 laufen.

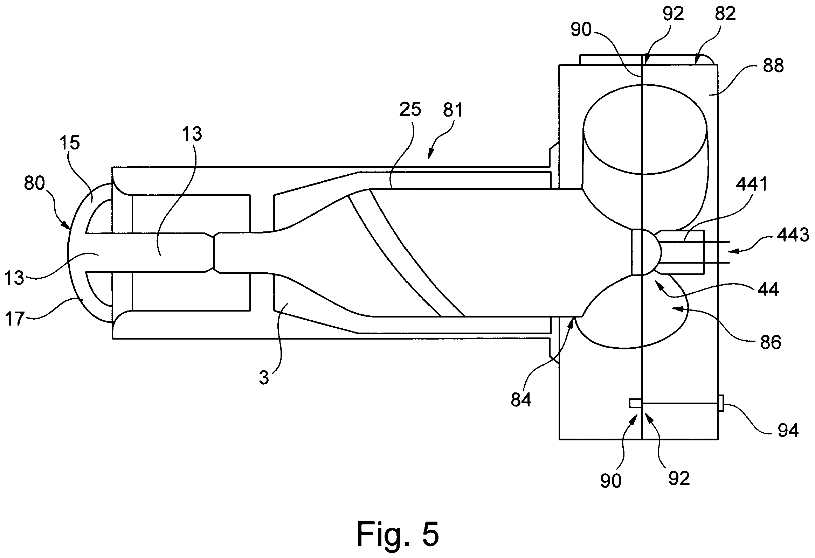

Eine alternative Ausführungsform ist zudem in der Fig. 5 gezeigt. Hierbei wird lediglich auf die alternative Ausführung des Einlassstators und der Streben eingegangen. In der Fig. 5 ist deutlich erkennbar, dass die Streben 15 und 17 sich bogenförmig stromaufwärts des Einlasses außerhalb desselben erstrecken und somit einen Dom 80 bilden, welcher selbst im Falle eines Anstoßens der stromaufwärtigen Seite des Doms an die Ventrikelwand eine weitere Blutförderung durch die Blutpumpe ermöglicht. Insbesondere bei einer physiologischen Regelung kann es zu derartigen Ansaugeffekten kommen. Die Käfigstruktur verhindert hier ein Ausbleiben der Blutförderung. Aufgrund der Bogenform der Streben kann der Stator 13 zudem weiter stromaufwärts gesetzt werden.

Die Spülbeschaufelung 76 und 78 kann beispielsweise dergestalt sein, dass die Spülbeschaufelung im Wesentlichen dünne Schaufeln sind, welche im kegelförmig zulaufenden Abschnitt 50 angeordnet sind. Dabei können die Schaufeln wie dargestellt zunächst eine Verlängerung des zylinderförmigen Abschnitts 48 bilden. Der Radius der Spülschaufeln fällt anschließend zum stromabwärtigen Ende des Strömungsbereichs hin schneller als der Radius des kegelförmigen Abschnitts 50 ab, so dass die Spülbeschaufelung ggf. bereits vor dem stromabwärtigen Ende des Abschnitts 50 in denselbigen läuft. Die Spülbeschaufelung kann wie in der Figur 2 gezeigt eine Krümmung entgegen der Laufradbeschaufelung aufweisen. In anderen Ausführungsbeispielen, wie z.B. der Figuren 3 und 4 verläuft die Spülbeschaufelung im Wesentlichen parallel zur Rotationsachse.

In der Figur 5 ist ferner eine stromaufwärtige Komponente 81 und eine stromabwärtige Komponente 82 gezeigt. Die stromaufwärtige Komponente 80 ist ein metallischer Grundkörper, welcher einen Teil des Pumpeneinlasses 3, insbesondere den Abschnitt 25 und den Übergang 84 zur Ringkammer 86 umfasst. Der Grundkörper kann dabei aus einem Werkstück gefertigt sein oder mittels eines generativen Verfahrens in einem Arbeitsgang gefertigt sein. Die stromabwärtige Komponente 82 umfasst einen Grundkörper 88, in welchem der stromabwärtige Abschnitt der Ringkammer geformt ist. Werden die beiden Komponenten 81, 82 aufeinandergelegt, wird zwischen den beiden Komponenten die Ringkammer 88 und ein Teil des Einlasses bzw. der Abschnitt 25 gebildet.

In der Figur 5 besitzt jede der beiden Komponenten 81 und 82 Anpressflächen 90 bzw. 92, welche zueinander korrespondieren und plan sind, da dies fertigungstechnisch gut umsetzbar ist. Über Verbindungselemente wie die Schrauben 94 werden die beiden Komponenten derart miteinander verbunden, dass das Innere der Pumpe gegenüber dem Körper abgedichtet ist, in welchem die Pumpe angeordnet wird. Die Anpressflächen können, wie in der Figur 5 dargestellt, ringförmig ausgebildet sein und vollflächig aufeinandergepresst werden. Beim Aufeinanderpressen der Komponenten wird zudem das hintere mechanische Lager 441 mit einem ersten Anpressdruck belegt, da der Rotor vor dem Verbinden der Komponenten in den Einlass eingelegt werden muss. Eine Feinjustierung der Lagerspannung kann entweder über das vordere Lager oder durch eine im Bereich des hinteren Lagers angeordnete Spannvorrichtung bzw. Einstellvorrichtung vorgesehen sein. Im vorliegenden Beispiel ist eine Justierschraube 443 zu sehen, welche den stromabwärtigen Teil 441 des hinteren Lagers auf den am Rotor angeordneten Lagerabschnitt anpresst.

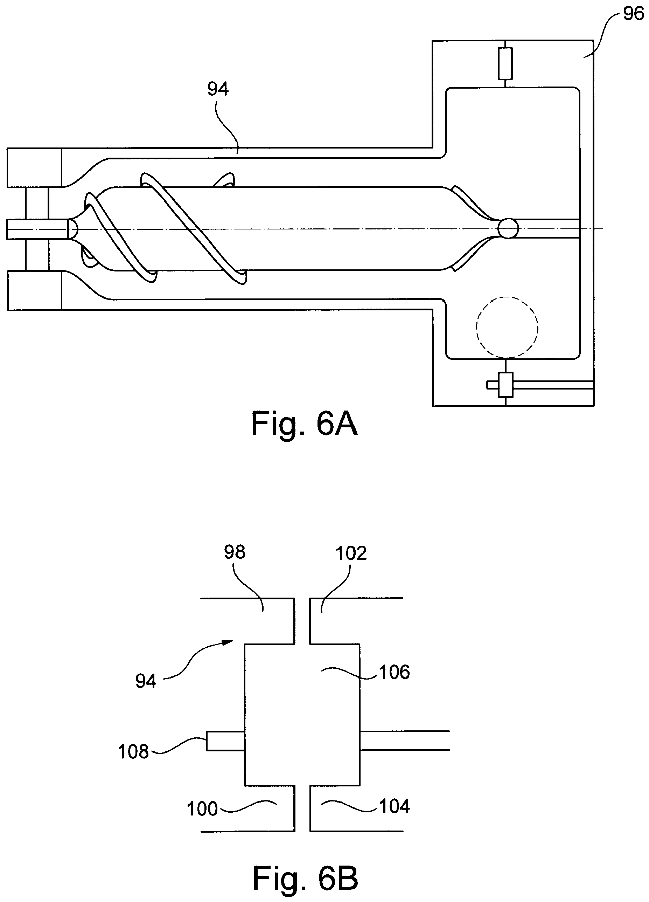

In einer alternativen Ausführungsform sind die Anpressflächen der beiden Komponenten 81 und 82 anders gestaltet. Im in der Figur 6 dargestellten Ausführungsbeispiel umfassen die beiden Komponenten 94 und 96 jeweils zwei Kragen. Die stromaufwärtige Komponente 94 besitzt einen die Ringkammer umlaufenden Kragen 98 und einen den äußeren Umfang umlaufenden Kragen 100. Ein Querschnitt der Kragen ist in der Figur 6B vergrößert dargestellt. Die stromabwärtige Komponente 96 besitzt entsprechende Kragen 102 und 104. Im aufeinanderliegenden Zustand der Komponenten 94 und 96 wird zwischen den aufeinanderliegenden Kragen 98 und 102 bzw. 100 und 104 ein Hohlraum 106 gebildet. Im Hohlraum können weitere Bohrungen 108 bzw. 110 vorhanden sein, welche zum Einführen der Verbindungselemente (z.B. Stifte oder Schrauben) verwendet werden können, um anschließend die beiden Komponenten aufeinanderzupressen. Alternativ kann auch nur eine der beiden Komponenten einen Kragen umfassen. In einigen Ausführungsbeispielen ist die Komponente ohne Kragen dann bevorzugt plan.

Anhand der Figuren 7A bis C soll die Wirkung des stromabwärtigen Profils einer Laufradbeschaufelung beschrieben werden. Dabei ist die in der Figur 7 C die zum Erzeugen einer Verwirbelung bevorzugte Beschaufelung. Dabei dient die Verwirbelung unter anderem auch der Wärmeabfuhr. Gleichzeitig sollte jedoch auch darauf geachtet werden, dass eine Blutschädigung vermieden wird. Eine Möglichkeit ist dabei, die Kanten derart zu wählen, dass kleinmaßstäbige Wirbel zugunsten größerer Wirbel vermieden werden. Weiterhin kann das stromabwärtige Kantenprofil mit einer eventuellen Spülbeschaufelung am stromabwärtigen Ende abgestimmt werden, um sowohl die Wärmeabfuhr als auch die Spülung des stromabwärtigen Lagers zu begünstigen.

In der Figur 7A ist der Rotor 110 mit einer Laufradbeschaufelung 112 dargestellt, welche zwei Schaufeln 114 und 116 umfasst (das stromabwärtige Ende der Schaufel 114 ist auf der Rückseite des Rotors nicht sichtbar, wohingegen nur das stromabwärtige Ende 118 der Schaufel 116 zu sehen ist). Verschiedene Varianten eines stromabwärtigen Endes einer Beschaufelung sind in den Figuren 7B und 7C dargestellt. Das zu fördernde Medium strömt die Schaufel dabei in der Richtung 120 an. Der Anstellwinkel \alpha des stromabwärtigen Endes 122 der Schaufel 124 ist identisch mit dem Anstellwinkel \alpha des stromabwärtigen Endes 132 der Schaufel 134. Im Gegensatz zur Schaufel 124 ist das Ende 132 der Schaufel 134 symmetrisch gestaltet, d.h. dass die stromzugewandte Schaufelkante 136 und die stromabgewandte Schaufelkante 138 im Wesentlich gleich lang sind und am Ende mit einem Krümmungsradius zusammenlaufen, welcher geringer ist als die Schaufelbreite 139. Aufgrund dieser Form des Endes werden Verwirbelungen 140 auf der stromabwärtigen Seite der Beschaufelung erzeugt, welche eine Drallströmung unterstützen können, die eine Spülung des stromabwärtigen Lagers begünstigt.

Die im Stand der Technik eingesetzten Schaufeln besitzen im Allgemeinen zwei Schaufelkanten 126 und 128, welche unterschiedlich lang sind und so Verwirbelungen vermeiden. Die in der Fig. 7B dargestellte Variante ist derart geformt, dass die Schaufelkante 126 am Ende einen Krümmungsradius besitzt, welcher deutlich breiter als die Schaufelbreite der Schaufel 124 ist und in einem spitzen Winkel mit der im Wesentlichen gerade verlaufenden Schaufelkante 128 zusammenläuft.

Die Figur 8 zeigt einen stromabwärtigen Abschnitt eines Rotors, welcher in einigen Ausführungsformen zum Einsatz kommt. Der Rotor 150 mit einer Rotationsachse besitzt einen zylinderförmigen Abschnitt 154, einen einer Hyperbel ähnlichen Abschnitt 156, einen weiteren zylinderförmigen Abschnitt 158 und einen kegelförmigen Abschnitt 160. Dabei sind die Abschnitte 154, 156, und zumindest Teil des Abschnitts 158 Teil der Rotorhülse, und der verbleibende Teil des Abschnitts 158 und der sich daran anschließende Teil des Abschnitts 160 in einer Ausnehmung 162 des Rotors gehalten und werden durch einen Lagerabschnitt 164 gebildet. Der Lagerabschnitt kann beispielsweise aus einem Hartstoff hergestellt sein. Im vorliegenden Beispiel umfasst der Lagerabschnitt den kegelförmigen Abschnitt sowie einen in der Ausnehmung eingelassenen Zapfen, welcher in der Ausnehmung beispielsweise mittels eines Fügemittels wie einem Kleber oder mittels einer Fassung, wie einer Zargen- oder eingeriebenen Fassung gehalten ist.

Das Profil des stromabwärtigen Abschnitts des Rotors 150 ist derart beschaffen, dass eine Spülung des Lagers am stromabwärtigen Ende des Lagerabschnitts 164 verbessert wird. Dabei ist der Abschnitt 156 entlang der Rotationsachse 152 länger als der Abschnitt 158, welcher wiederum kürzer als der Abschnitt 160 ist. Diese Längenverhältnisse unterstützen die Verbesserung des Ausspüleffekts.

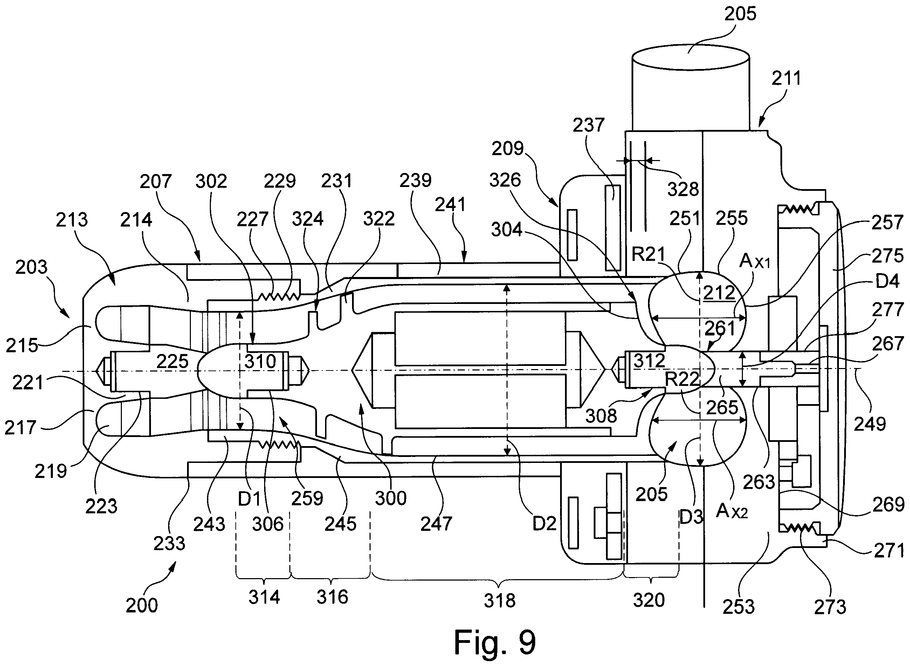

In der Figur 9 wird eine alternative Ausführungsform einer Pumpe 200 dargestellt. Die Pumpe 200 weist zahlreiche Ähnlichkeiten mit der Pumpe, wie sie in der Figur 2 dargestellt ist, auf.
Die Pumpe 200 besitzt einen Einlass 203 und einen Auslass 205. Ferner ist ein Außengehäuse 207 vorhanden, an dessen stromabwärtigem Ende ein Konnektor 209 zur Befestigung an einem Nahtring angeordnet ist. Ferner ist die Außenwand 211 der Pumpe erkennbar, welche den Auslass und die Spiralkammer 212 beherbergt.

Innerhalb des Außengehäuses 207 ist eine Einlasshülse 213 angeordnet, welche eine Außenwand 214 mit einer sich zwischen zwei Streben 215 und 217 liegenden Einlassöffnung 219 besitzt. Die Streben 213 und 215 bedecken je nach Ausführungsform zwischen einem 1/10 und einem 1/3 der durch den Innendurchmesser der Außenwand definierten Einlassfläche. Dementsprechend verbleibt die zwischen 2/3 und 9/10 der Einlassfläche als Einlassöffnung 219. Ferner halten die Streben 215 und 217 einen Stator 221, welcher sich stromabwärts erstreckt und eine Ausnehmung 223 zur Aufnahme einer Lagerkalotte 225 besitzt. Die Einlasshülse besitzt einen Innendurchmesser D1, welcher bei einer pädiatrischen Pumpe 5 - 12 mm beträgt; bei einer Erwachsenenpumpe beträgt D17 -14 mm und weitet dann auf D2 = 12 - 20 mm auf. Eine solche Aufweitung ist auch bei einer pädiatrischen Pumpe möglich, aber nicht zwingend. Die Gesamtlänge der Einlasshülse (ohne Nahtring) beträgt bei einer pädiatrischen Pumpe 15 - 30 mm, bei einer Erwachsenenpumpe 25 - 50 mm. Dazu kommen bei beiden Pumpen noch je 2 - 8 mm Höhe der Streben über der Einlasskanüle. Im stromabwärtigen Bereich ist ein Gewinde 227 angeordnet, welches mit einem korrespondierenden Gewinde 229 des Innenrohrs 231 verschraubt ist. Die Einlasshülse ist aus einem Werkstoff wie Titan oder einem anderen biokompatiblen Material, wie z. B. biokompatiblem Stahl oder Keramik, hergestellt.

Im Außengehäuse 207 befindet sich eine Öffnung 233, welche durch die eingesteckte Einlasshülse vollständig abgedeckt ist. Auf diese Weise ergibt sich zwischen der Innenwand des Außengehäuses 207, der Einlasshülse 213 und dem Innenrohr 231 eine Hohlraum 235, in welchem sowohl Teile der Pumpenelektronik 237, wie Steuerungskomponenten und Kabel, angeordnet sein können als auch die Wicklungen 239 des Motorstators 241.

Das Innenrohr 231 umfasst einlassseitig einen stromaufwärtigen Abschnitt 243 mit einem zylinderförmigen Hohlraum. Der Hohlraum besitzt ebenfalls einen Innendurchmesser D1 und schließt bündig mit der Innenwand der Einlasshülse ab. Stromabwärts schließt sich ein Abschnitt 245 an den Abschnitt 243 an, wobei der Innendurchmesser im Abschnitt 245 stetig vom Wert D1 zum Wert D2 zunimmt. An diesen sich aufweitenden Abschnitt 245 schließt sich der zylinderförmige Abschnitt 247 an, welcher einen Hohlraum mit Innendurchmesser D2 definiert. Entlang der Rotationsachse 249 besitzt der Abschnitt 243 eine Länge von 1 - 5 mm, der sich aufweitende Abschnitt 245 eine Länge von 5 -15 mm und der Abschnitt 247 eine Länge von 15 - 20 mm auf. Der zylinderförmige Abschnitt 247 geht in die Spiralkammer 212 über. Der Innenraum der Spiralkammer 212 wird unter anderem durch die Innenwand 251 und die Innenwand 255 der Abdeckplatte 253 begrenzt. Der Innenraum weitet sich vom Durchmesser D2 zunächst zum Durchmesser D3 auf, und nimmt stromabwärts anschließend auf den Durchmesser D4 ab.

An das Innenrohr 231 schließt sich eine Abdeckplatte 253 an, deren Innenwand 255 bündig mit der Innenwand 251 des Innenrohrs abschließt. Die Innenwände 251 und 255 formen so die Spiralkammer 212. Die Innenwand 255 der Abdeckplatte 253 weist zudem eine sich in der stromaufwärtigen Richtung erstreckende Erhöhung 257 mit dem Durchmesser D4 auf, deren Achse mit der Rotationsachse 249 zusammenfällt. Insgesamt wird der Strömungsbereich 259, welcher sich zwischen dem Einlass 203 und dem Auslass 205 erstreckt also durch die Innenwände der Einlasshülse 215, des Innenrohrs 231 und der Abdeckplatte 253 begrenzt. Der Durchmesser D2 setzt sich aus den Radii R21 und R22 zusammen, wobei der Radius R22 größer als der Radius R21 ist, d.h. der Radius der Spiralkammer nimmt zum Auslass hin zu. Im Bereich der Spiralkammer 212, welcher in der Figur 9 oberhalb der Rotationsachse 249 eingezeichnet ist, ist zudem die maximale axiale Erstreckung Ax1 der Spiralkammer kleiner als die maximale axiale Erstreckung Ax2 der Spiralkammer im Bereich unterhalb der Rotationsachse 249. Die axiale Erstreckung der Spiralkammer nimmt zumindest stromabwärts betrachtet rechtsdrehend von der Erstreckung Ax1 zur Erstreckung Ax2 zu. D.h. die Spiralkammer weitet sich nicht nur radial, sondern auch axial betrachtet, rechtsdrehend auf. Diese zweidimensionale Aufweitung wird auch als Schneckenkammer bezeichnet.

In eine an der Position 261 (beispielsweise mittig) der Erhöhung liegende Ausnehmung 263 ist eine Lagerkalotte 265 angeordnet, welche einen Teil des stromabwärtigen Lagers bildet. Im vorliegenden Ausführungsbeispiel ist der Durchmesser der Lagerkalotte quer zur Rotationsachse kleiner als der Durchmesser der Lagerkalotte 225. In anderen Ausführungsbeispielen kann der Durchmesser gleich oder größer sein. Die Lagerkalotte 265 wird am stromabwärtigen Ende in einer Metallhülse 267 gehalten. Die Metallhülse weist ein Gewinde auf, welches zum einen mit der Rückwand 269 der Abdeckplatte 255 verschraubt werden kann. Die Rückwand 269 besitzt zudem einen Kragen 271 mit einem Gewinde 273, in welches eine Dichtplatte 275 eingeschraubt werden kann. Die Dichtplatte 275 ist derart angeordnet, dass die Achse der Dichtplatte konzentrisch mit der Rotationsachse 249 der Pumpe liegt. Im Bereich der Achse besitzt die Dichtplatte 275 ein weiteres Gewinde 277, welches ebenfalls mit dem Gewinde der Metallhülse korrespondiert. Durch Verschrauben der Dichtplatte kann so die Länge, mit welcher die Lagerkalotte 265 in den Strömungsbereich ragt, variiert werden. Mit Hilfe der Dichtplatte 275 kann beispielsweise später die Lagerspannung justiert werden.

Die Einlasshülse 215, das Innenrohr 231 und die Abdeckplatte 255 sind vorzugsweise jeweils einstückig ausgebildet, da dies die Montage des Rotors 263 innerhalb der Pumpe stark vereinfacht. Geeignete Materialien sind hierbei Titan, Titanlegierungen oder medizinische Stähle. Die gilt auch für die Dichtplatte.

Der Rotor 300 besitzt an seinem stromaufwärtigen Ende 302 und an seinem stromabwärtigen Ende 304 Ausnehmungen 306 bzw. 308, in welche die zu den statischen Lagerelementen 225 bzw. 265 korrespondierenden Lagerelemente 310 bzw. 312. Die Lagerelemente 310 bzw. 312 haben eine Pilzform mit einem zylinderförmigen Abschnitt und einem Pilzhutabschnitt, welcher an seinem jeweiligen Ende mit dem Kalottensegment 225 bzw. 265 korrespondiert. Die Lagerelemente 310 bzw. 312 weisen Diamant, Diamantähnliche Werkstoffe oder andere Hartstoffe wie SiC auf oder bestehen aus diesen. Bezüglich der Lagerelemente wird auf die PCT/EP2017/074796 verwiesen, welche vollumfänglich zum Bestandteil dieser Anmeldung gemacht wird.

Der Rotor 300 besitzt eine Spindelform, welche sich in vier Abschnitte aufteilen lässt: einen zylinderförmigen Abschnitt 314, einen sich aufweitenden Abschnitt 316, einen weiteren zylinderförmigen Abschnitt 318, und einen abnehmenden Abschnitt 320. Eine Beschaufelung 322 ist im Wesentlichen im Abschnitt 316 angeordnet und erstreckt sich im vorliegenden Ausführungsbeispiel bis in den Beginn des Abschnitts 318.

Der Abschnitt 314 besitzt einen Durchmesser RD1, der Abschnitt 318 einen Durchmesser RD2. Die Querschnittsfläche des Netto-Strömungsbereichs, durch welche die zu pumpende Flüssigkeit vom Einlass 203 zur Spiralkammer 212 gefördert werden kann, ist jeweils (D1^2-RD1^2)*pi/4 bzw. (D2^2-RD^2)*pi/4. Der Durchmesser des Rotors nimmt im Bereich des Abschnitts 316 entlang der Rotationsachse 249 schneller zu als der entsprechende Durchmesser des Innenrohrs 231. Der Abstand zwischen dem radial äußersten Abschnitt 324 der Beschaufelung 322 und dem Innenrohr 231 bleibt entlang der Beschaufelung im Wesentlichen konstant, so dass die radiale Höhe der Beschaufelung im Abschnitt 316 stromabwärts hin abnimmt. Die Dicke der Beschaufelung entlang der Rotationsachse ist im vorliegenden Ausführungsbeispiel im Wesentlichen unverändert. Diese Form begünstigt wiederum den Druckaufbau entlang des Rotors durch die Verzögerung der Strömung. Bezüglich der Form des stromabwärtigen Endes des Beschaufelung wird auf die Figuren 7 und 8 verwiesen.

Eine Spülung des stromaufwärtigen Lagers kann beispielsweise mit der sich zwischen dem Abschnitt 324 und dem Innenrohr 231 ausbildenden, stromaufwärts gerichteten Sekundärströmung beeinflusst werden. Anders als im stromaufwärtigen Abschnitt 316 des Rotors 300, befindet sich zwischen dem Abschnitt 318 und dem Abschnitt 320 eine Kante 326, weiche die Verringerung des Durchmessers in stromabwärtiger Richtung markiert. Die Verringerung des Durchmessers des Rotors an der Kante 326 beginnt dabei noch im Abschnitt 247 des Innenrohrs. Die Aufweitung des Durchmessers im Innenrohr beginnt erst mit leichtem stromabwärtigen Versatz 328.

Die in der Figur 9 dargestellte Pumpe 200 kann auf besonders einfache Art und Weise montiert werden. Zunächst wird der Einlasshülse 213 mit dem Innenrohr 231 verschraubt und im Außengehäuse 207 angeordnet. Anschließend wird der Rotor 300 in das Innenrohr 231 eingeführt und danach die Abdeckplatte 253 aufgesetzt und mit dem Innenrohr 231 verbunden. Danach wird die Spannplatte 275 montiert, und die gewünschten Lagerspannungen können eingestellt werden.

## Patentansprüche

1. Blutpumpe, umfassend:
ein Gehäuse mit einer Gehäuseachse, einem stromaufwärtigen Einlass und einem stromabwärtigen Auslass und einem zwischen dem Einlass und dem Auslass ausgebildeten Strömungsbereich;
einen im Gehäuse angeordneten spindelförmigen Rotor mit einer Beschaufelung;
wobei der spindelförmige Rotor einen stromaufwärtigen Einlassabschnitt, einen Zylinderabschnitt, und einen stromabwärtigen Auslassabschnitt mit einer Rotationsachse aufweist;
wobei der spindelförmige Rotor stromabwärts zumindest teilweise mechanisch gelagert ist;
wobei die Beschaufelung des Rotors zumindest eine sich im stromaufwärtigen Einlassabschnitt und dem Zylinderabschnitt angeordnete Laufradbeschaufelung mit einer sich entlang der Rotationsachse ersten Krümmungsrichtung aufweist;
wobei der Strömungsbereich stromabwärts des Einlasses eine entlang der Gehäuseachse gelegene Auslasskammer mit einem vergrößerten Querschnitt aufweist und eine durch den stromabwärtige Auslass gegebene Ausströmrichtung transversal zur Gehäuseachse liegt; und
das Gehäuse ferner derart ausgebildet ist, dass der Strömungsbereich zwischen dem stromaufwärtigen Abschnitt und dem zylinderförmigen Abschnitt entlang der Gehäuseachse einer Vergrößerung des Querschnitts erfährt.

2. Blutpumpe nach Anspruch 1, wobei der stromabwärtige Abschnitt des Rotors in der Auslasskammer liegt.

3. Blutpumpe nach einem der vorhergehenden Ansprüche 1 bis 2, wobei das Gehäuse in der Auslasskammer einen koaxial zur Gehäuseachse verlaufenden und sich stromaufwärtig erstreckenden Auslassstator umfasst, welcher einen Teil des stromabwärtigen Lagers bildet.

4. Blutpumpe nach einem der vorhergehenden Ansprüche, wobei ein Einlassstator mittels Streben am Gehäuse angeordnet ist.

5. Blutpumpe nach Anspruch 4, wobei ein stromabwärtiger Abschnitt des Einlassstators einen Teil einer mechanischen Lagerung für den Rotor bildet.

6. Blutpumpe nach einem der Ansprüche 4 oder 5, wobei die Streben außerhalb des Einlasses angeordnet sind.

7. Blutpumpe nach einem der vorhergehenden Ansprüche, wobei der Rotor entlang der Rotationsachse stromaufwärts und stromabwärts mechanisch gelagert ist.

8. Blutpumpe nach einem der vorhergehenden Ansprüche, wobei der Rotor ferner eine im stromabwärtigen Auslassabschnitt angeordnete Spülbeschaufelung mit einer der ersten Krümmungsrichtung entgegengesetzten Krümmungsrichtung und/oder eine Rillenstruktur zum Auswaschen der stromabwärtigen mechanischen Lagerung umfasst.

9. Blutpumpe nach Anspruch 8, wobei die Spülbeschaufelung bzw. die Rillenstruktur nur am stromabwärtigen Abschnitt des spindelförmigen Rotors angeordnet ist.

10. Blutpumpe nach einem der vorhergehenden Ansprüche, wobei der stromabwärtige Abschnitt und/oder stromaufwärtige Abschnitt des spindelförmigen Rotors kegelförmig oder glockenförmig ist.

11. Blutpumpe nach einem der vorhergehenden Ansprüche, wobei die mechanische Lagerung ein Diamantlager, vorzugsweise ein eine Diamantkugel und eine Diamantkalotte umfassendes Diamantlager, oder eine Hartstofflager umfasst.

12. Blutpumpe nach einem der vorhergehenden Ansprüche, wobei der Rotor eine diamantartige Beschichtung besitzt.

13. Blutpumpe nach einem der vorhergehenden Ansprüche, wobei sich der stromabwärtige Abschnitt des Rotors direkt an den zylinderförmigen Abschnitt anschließt und sich stromabwärts verjüngt.

14. Blutpumpe nach einem der vorhergehenden Ansprüche, wobei in einer Wand des Gehäuses ein Drucksensor zur Messung eines Ventrikeldrucks im Strömungsbereich vorhanden ist.

15. Blutpumpe nach einem der vorhergehenden Ansprüche, wobei mindestens eine Spannvorrichtung oder Einstellvorrichtung zum Justieren der mindestens einen mechanischen Lagerung vorhanden ist.
